# EUROPÄISCHE PATENTSCHRIFT

(11) **EP 1 162 953 B1**
(45) Veröffentlichungstag und Bekanntmachung des Hinweises auf die Patenterteilung: **03.03.2004**
(21) Anmeldenummer: 00914147.4
(22) Anmeldetag: 17.03.2000
(51) Int. Cl.: A61K 9/20

(54) **MECHANISCH STABILE PHARMAZEUTISCHE DARREICHUNGSFORMEN, ENTHALTEND FLÜSSIGE ODER HALBFESTE OBERFLÄCHENAKTIVE SUBSTANZEN**
MECHANICALLY STABLE PHARMACEUTICAL PRESENTATION FORM CONTAINING LIQUID OR SEMI-SOLID SURFACE-ACTIVE SUBSTANCES
FORMES D'ADMINISTRATION PHARMACEUTIQUES MECANIQUEMENT STABLES, QUI CONTIENNENT DES SUBSTANCES TENSIOACTIVES LIQUIDES OU SEMI-SOLIDES

(30) Priorität: 25.03.1999 DE 19913692
(43) Veröffentlichungstag der Anmeldung: 19.12.2001
(73) Patentinhaber: BASF AKTIENGESELLSCHAFT, 67056 Ludwigshafen (DE)
(72) Erfinder: ROSENBERG, Jörg, D-67158 Ellerstadt (DE); BERNDL, Gunther, D-67273 Herxheim (DE); LIEPOLD, Bernd, D-68161 Mannheim (DE); BREITENBACH, Jörg, D-68199 Mannheim (DE)
(74) Vertreter: Riedl, Peter, Dr.
(86) Internationale Anmeldenummer: PCT/EP2000/002381
(87) Internationale Veröffentlichungsnummer: WO 2000/057854

(56) Entgegenhaltungen:
- EP-A- 0 272 336
- EP-A- 0 551 820

## Beschreibung

Die vorliegende Erfindung betrifft mechanisch stabile pharmazeutische Darreichungsformen zur peroralen Verabreichung, enthaltend einen oder mehere Wirkstoffe, mindestens einen thermoptastisch Verarbeitbaren matrixbildenden Hilfsstoff, der ausgewählt ist unter Homo- oder Copolymeren des N-Vinylpyrnolidons, Acrylatpolymeren vom Eudragit-Typ und Cellulosederivaten, und mehr als 10 und bis zu 40 Gew.-% einer obernachenaktiven Substanz mit einem HLB-Wert von 2 bis 18, weiche bei 20°C flüssig ist oder einen Tropfpunkt im Bereich von 20 bis 50°C aufweist, erhältlich durch Vermischen der Einsatzstoffe in der Schmelze ohne Zugabe von Lösungsmitteln und anschließender Formgebung. Weiterhin wurde ein Verfahren zur Herstellung solcher Formen gefunden.

Die Herstellung pharmazeutischer Zubereitungen nach dem Schmelzextrusionsverfahren ist an sich bekannt. So ermöglicht das beispielsweise in der EP-A 240 904 oder der EP-A 240 906 beschriebene Verfahren durch gezielte Auswahl bzw. definierte Abmischungen der eingesetzten Hilfsstoffe eine gezielte Steuerung der Eigenschaften der herzustellenden Formulierungen.

Durch Auswahl geeigneter Matrixpolymere lassen sich zum Beispiel Zubereitungen herstellen, die den Wirkstoff über einen längeren Zeitraum kontinuierlich freisetzen. Andererseits kann es erwünscht sein, z.B. bei Schmerzmitteln eine rasche Auflösung mit schneller Freisetzung des Wirkstoffs zu erzielen. Sowohl für die Herstellung von schnell freisetzenden wie auch von langsam freisetzenden Zubereitungsformen hat sich das Schmelzextrusionsverfahren als geeignet erwiesen.

Eine Grundvoraussetzung ist allerdings ein ausreichendes Auflösungsvermögens des wirkstoffs im wäßrigen Milieu des Verdauungstrakts. Die Resorption des Wirkstoffs ist nur dann möglich, wenn dieser gelöst vorliegt, da nur gelöste Wirkstoffe die Darmwand passieren können. Im Falle schwerlöslicher Wirkstoffe kann dies zu ungenügender Resorption und damit verbundener niedriger Bioverfügbarkeit führen.

Es hat nicht an Versuchen gefehlt, die Bioverfügbarkeit schwerlöslicher Wirkstoffe zu verbessern (vgl. R. Voigt; "Pharmazeutische Technologie", Verlag Ullstein Mosby, 7. Auflage, 1993, Seiten 80-85). Insbesondere die Herstellung von Coevaporaten oder sogenannten festen Dispersionen, in denen der Wirkstoff molekulardispers in einer Hilfsstoffmatrix verteilt vorliegt, hat sich häufig als vorteilhaft für die Erhöhung der Bioverfügbarkeit erwiesen. Bei der Auflösung der Arzneiform im Körper kann der Wirkstoff aus solchen festen Dispersion direkt und ohne Aufbringen von Solvatationsenergie molekular freigesetzt werden.

Einen positiven Einfluß auf die Bioverfügbarkeit des Wirkstoffs hat die Anwendung von festen Dispersionen aber nur, wenn der Wirkstoff auch schnell resorbierbar ist. Ist der Resorptionsvorgang aber langsam, kommt es zu einer Rekristallisation des schwerlöslichen Wirkstoffs im wäßrigen Milieu des Darmlumens, da bei der Auflösung der Arzneiform eine übersättigte Wirkstofflösung entstehen kann. Aus diesem Grund sind auch mit festen Dispersionen häufig nur unbefriedigende Bioverfügbarkeiten zu erzielen.

Oft scheitert die ausreichende Resorption des Wirkstoffs auch daran, daß der Wirkstoff zu langsam aus der Tablette freigesetzt wird. Die Resorption in die Blutzirkulation findet für den überwiegenden Teil aller Wirkstoffe in den oberen Dünndarm-Abschnitten statt, d.h. relativ kurz nach Passage des Magens. wirkstoffe, die in diesem Bereich des Dünndarms noch nicht ausreichend solubilisiert wurden, können nur noch begrenzt resorbiert werden.

Zur Erzielung optimaler Resorptionsraten ist es daher entscheidend, insbesondere bei schwerlöslichen, leicht kristallisierenden Wirkstoffen eine schnelle und genügend lang andauernde Solubilisierung im wäßrigen Milieu des Verdauungstrakts zu erreichen, ohne daß dabei eine Rekristallisation eintritt.

Hierzu bietet sich der Zusatz oberflächenaktiver Substanzen an. Die zugabe von oberflächenaktiven Substanzen zu Formulierungen schwerlöslicher Wirkstoffe ist an sich allgemein bekannt.

Aus der US-A 5,834,472 ist beispielsweise bekannt, daß durch Mitverwendung einer nichtionischen oberflächanaktiven Substanz die Bioverfügbarkeit eines Antifungicides verbessert werden kann.

Da die meisten der oberflächenaktiven Substanzen bei Raumtemperatur aber flüssig oder halbfest sind, werden daher bisher meist flüssig-ölige oder halbfeste Zubereitungen hergestellt, die dann in Hart- oder Weichgelatine-Kapseln abgefüllt werden. Jedoch treten bei Weichgelatine-Kapseln häufig Wechselwirkungen zwischen Hilfsstoffen und der Gelatinehülle der Kapsel auf, die zum vorzeitigen Auslaufen der Kapsel führen.

Auch der Einsatz der oberflächenaktiven Substanzen in Tabletten-Formulierungen ist nicht ohne weiteres möglich, da die flüssigen oder halbfesten oberfächenaktiven Substanzen die Verpreßbarkeit beim konventionellen Tablettierprozeß verhindern, insbesondere dann, wenn zur Solubilisierung des Wirkstoffs größere Mengen an oberflächenaktiven Substanzen im Bereich von mehr als 10 Gew.-% benötigt werden.

Der vorliegenden Erfindung lag daher die Aufgabe zugrunde, mechanisch stabile feste Zubereitungsformen für die perorale Anwendung zu finden, die insbesondere bei schwerlöslichen Wirkstoffen zu einer schnellen und trotzdem langanhaltenden Solubilisierung nach deren Liberation aus der Arzneiform dienen können.

Demgemäß wurden die eingangs definierten pharmazeutischen Zubereitungsformen und ein Verfahren zu deren Herstellung gefunden.

Als Wirkstoffe können prinzipiell alle pharmazeutischen Substanzen für den Human- wie den Veterinärbereich sowie Wirkstoffe, die in der Nahrungsmittelergänzung Anwendung finden, eingesetzt werden.

Als geeignete Wirkstoffe kommen vor allem Immunsuppressiva, Protease-Inhibitoren, Reverse-Transskriptase-Inhibitoren, Cytostatica oder Antimykotika in Betracht, weiterhin auch ZNS-aktive Wirkstoffe oder Dihydropyrimidinderivate.

Insbesondere lassen sich erfindungsgemäß schwerlösliche oder schwer bioverfügbare Wirkstoffe formulieren. Schwer löslich bedeutet, daß die Löslichkeit im wäßrigen Milieu unter 1 mg/ml liegt. Solche Wirkstoffe werden gemäß USP XXII, Seite 8, auch als kaum löslich oder praktisch unlöslich bezeichnet. Schwerlösliche Wirkstoffe sind beispielsweise Esupron, Nifedipin, Ciclosporin oder Taxol.

Als oberflächenaktive Substanzen kommen vorzugsweise niedermolekulare Substanzen in Betracht, die einen HLB-Wert (HLB - Hydrophilic Lipophilic Balance) aufweisen, und bei 20°C flüssig sind oder einen Tropfpunkt im Bereich von über 20°C bis 50°C aufweisen, bevorzugt von bis zu 40°C. Bevorzugt werden Substanzen mit einem HLB-Wert von 7 bis 18, besonders bevorzugt 10 bis 15.

Geeignete oberflächenaktive Substanzen sind beispielsweise gesättigte und ungesättigte polyglykolisierte Glyceride, halbsynthetische Glyceride, Fettsäurester oder Ether von Fettsäurealkoholen, sofern sie die oben angegebenen Eigenschaften aufweisen.

Insbesondere eignen sich die entsprechenden Sorbitanfettsäureester oder ethoxilierte Sorbitanfettsäureester
wie beispielsweise Polyoxyethylen(20)sorbitanmonolaurat,
Polyoxyethylen(20)sorbitanmonopalmitat,
Polyoxyethylen(20)sorbitanmonostearat,
Polyoxyethylen(20)sorbitanmonooleat,
Polyoxyethylen(20)sorbitantristearat,
Polyoxyethylen(20)sorbitantrioleat,
Polyoxyethylen(4)sorbitanmonoscearat,
Polyoxyethylen(4)sorbitanmonolaurat oder
Polyoxyethylen(4)sarbztanmonooleat.
weiterhin eignen sich Macrogol-6-Cetylstearylether oder Macrogol-25-Cetylstearylether.

Besonders bevorzugt sind Polyoxyethylenglycerolricinolat-35, Polyoxyethylenglyceroltrihydroxystearat-40, PEG-660-12-Hydroxystearinsäure (Polyglykolester der 12-Hydroxystearin-säure (70 mol-%) mit 30 mol-% Ethylenglykol).

Die oberflächenaktiven Substanzen sind in den Zubereitungen in Mengen von mehr als 10 Gew.-%, bezogen auf das Gesamtgewicht der Zubereitung, und bis zu 40 Gew.-% enthalten, bevorzugt 15 bis 25 Gew.-% und besonders bevorzugt 20 bis 25 Gew.-%.

Weiterhin enthalten die erfindungsgemäßen Zubereitungen noch mindestens einen thermoplastisch verarbeitbaren Matrix-Hilfsstoff. Als matrixbildende Hilfsstoffe eignen sich vor allem wasserlösliche pharmazeutisch akzeptable Polymere oder Zuckeralkohole oder Gemische davon, sofern sie sich unzersetzt schmelzen lassen.

Pharmazeutisch akzeptable Polymere sind insbesondere Homo- und Copolymere des N-Vinylpyrrolidons wie Polyvinylpyrrolidon mit K-Werten nach Fikentscher von 12 bis 100, insbesondere K 17 bis K 30, oder Copolymere mit Vinylcarbonsäureestern wie vinylacetat oder vinylpropionat, beispielsweise Copovidone (VP/VAc-60/40).

Weiterhin eignen sich Polyvinylalkohol oder Polyvinylacetat, welches auch verseift oder teilverseift sein kann, oder AcrylatPolymere vom Eudragit-Typ.

Weiterhin eignen sich Cellulosederivate wie Hydroxyalkylcellulosen, beispielsweise Hydroxypropylcellulose, oder im Falle gewünschter langsamerer Freisetzung Hydroxyalkyl-alkylcellulosen, die in Wasser quellen, beispielsweise Hydroxypropyl-methylcellulose (HPMC), bevorzugt solche mit Methoxy-Substitutionsgraden im Bereich von 22 % und Hydroxypropoxy-Substitutionsgraden im Bereich von 8 %, besonders bevorzugt HPMC-Typen mit Viskositäten von 4000 mPas, 15000 mPas oder 100000 mPas, gemessen bei 20°C in 2 gew.-%iger wäßriger Lösung. Geeignet sind auch HPMCtypen mit Methoxy-Substitutionsgraden im Bereich von 28 bis 29 % und Hydroxypropoxy-Substitutionsgraden im Bereich von 5 bis 8.5 %.

Ebenso eignen sich schmelzbare Zuckeralkohole wie beispielsweise Sorbit, Maltit, Isomalt, Mannit, Xylit, Erythrit oder Mischungen daraus. Bevorzugt sind Maltit, Mannit, Xylit oder Isomalt.

Geeignete matrixbildende Polymere sind auch Polyethylenglykole mit Molekulargewichten im Bereich von 1000 bis 20000000 Dalton, bevorzugt 4000 bis 10000 Dalton.

Weiterhin können die Zubereitungen noch übliche pharmazeutische Hilfsstoffe wie Aromen, Antioxidantien, Kieselsäuren, Trennmittel oder Farbstoffe in den hierfür üblichen Mengen enthalten.

Die Herstellung der erfindungsgemäßen Zubereitungen erfolgt über ein Schmelzeverfahren. Bevorzugt wird das Verfahren ohne Zugabe von Lösungsmitteln durchgeführt.

Das Schmelzeverfahren wird in einem Kneter oder einem Schneckenextruder durchgeführt. Geeignete Kneter sind beispielsweise Kneter der Firmen Haake oder Farrell.

Bevorzugt erfolgt die Herstellung der Schmelze in einem Schneckenextruder, besonders bevorzugt einem Doppelschneckenextruder mit und ohne Knetscheiben oder ähnlichen Mischelementen. Besonders bevorzugt sind gleichsinnig drehende Doppelschneckenextruder.

In Abhängigkeit von der Zusammensetzung erfolgt die Verarbeitung im allgemeinen bei Temperaturen von 40°C bis 260°C, bevorzugt 50 bis 200°C.

Die Einsatzstoffe können dem Extruder oder Kneter einzeln oder als Vormischung zugeführt werden. Die Zugabe erfolgt bevorzugt in Form von pulverförmigen oder granulierten Vormischungen. So kann die flüssige oder ölige oberflächenaktive Substanz zuvor mit einem anderen Einsatzstoff zu einem rieselfähigen Granulat vermengt werden. Eine Zugabe der oberflächenaktiven Substanz in flüssiger Form, beispielsweise über Flüssigkeitspumpen, die bei halbfesten Substanzen vorzugsweise beheizt werden, ist ebenfalls möglich.

Man kann auch zunächst den Wirkstoff in der oberflächenaktiven Substanz lösen und diese Mischung dann mit dem Polymer granulieren. Dabei muß der Wirkstoff selbst nicht schmelzen.

Bei temperaturempfindlichen Wirkstoffen kann es sich auch empfehlen, zunächst die anderen Einsatzstoffe aufzuschmelzen und dann erst den Wirkstoff zuzugeben.

Die Einsatzstoffe werden demgemäß gemeinsam zu einer Schmelze verarbeitet, welche durch Eintragen mechanischer Energie, insbesondere in Form von Scherkräften, zu einer homogenen Masse verarbeitet wird.

Die homogene Schmelze wird anschließend durch eine Düse oder eine Lochplatte extrudiert und der Formgebung unterworfen. Dies kann durch Abschlag des strangförmig austretenden Extrudats mit den üblichen Abschlagtechniken erfolgen, beispielsweise mit Hilfe rotierender Messer oder durch Druckluftabschlag, wobei Pellets oder Granulate entstehen. Weiterhin kann die Formgebung wie in der EP-A 240 906 beschrieben erfolgen, indem das strangförmig austretende Extrudat zwischen zwei gegenläufig rotierende Kalanderwalzen geführt und direkt zu Tabletten ausgeformt wird. Ebenso kann die Schmelze über den offenen Extruderkopf ausgefahren werden und nach Erstarren gegebenenfalls noch gemahlen werden oder durch geeignete Granuliergeräte wie Walzenstühle oder Kompaktiereinheiten weiterverarbeitet werden.

Granulate oder Pellets können dann in konventionellen Tablettenpressen zu Tabletten verarbeitet werden. Es ist auch möglich, die durch Kalandrierung zunächst bereits in Form von mechanisch stabile Tabletten erhaltenen Zubereitungen einem Mahlvorgang zu unterwerfen und dann auf konventionelle Weise zu Tabletten zu verpressen. Gewünschtenfalls können die Tabletten dann mit einem üblichen Überzug versehen werden.

Überraschenderweise lassen sich erfindungsgemäß Tabletten erhalten, die trotz eines hohen Anteils an flüssigen oder halbfesten oberflächenaktiven Substanzen eine gute mechanische Stabilität aufweisen und nicht zur Klebrigkeit oder zum Erweichen neigen. Auf eine Abfüllung in Kapseln kann aufgrund der guten Formstabilität der Zubereitungen erfindungsgemäß verzichtet werden.

Die resultierenden Arzneiformen enthalten den Wirkstoff als amorphe Einbettung. Bevorzugt entstehen feste Dispersionen, in denen der Wirkstoff molekulardispers verteilt vorliegt. Die erfindungsgemäßen Arzneiformen ermöglichen es, auch schwerlösliche Wirkstoffe ausreichend zu solubilisieren bzw. in wäßrigem Medium stabil zu dispergieren.

Die erfindungsgemäßen zubereitungen bilden nach Auflösen in wäßrigem Medium, insbesondere bei pH-Werten von 1, für mindestens eine Stunde ein stabiles Solubilisat oder eine stabile Dispersion, in der der Wirkstoff bevorzugt nicht-kristallin vorliegt.

### Beispiel 1

50 g einer Pulvermischung aus 40 Gew.-% Esupron, 35 Gew.-% Polyvinylpyrrolidon K17 (PVP und 25 Gew.-% Polyoxyethylenglyceroltrihydroxystearat-40 als oberflächenaktive Substanz wurde derart hergestellt, daß zunächst eine pulvrige Vormischung aus Esupron und dem PVP hergestellt wurde, in die bei 20°C die oberflächenaktive Substanz untergemischt wurde, bis ein homogenes Granulat entstanden war.

### Beispiel 2

Das gemäß Beispiel 1 erhaltene Granulat wurde bei einer Temperatur von 100°C in einem beheizbaren Kneter (Firma Haake) zu einer homogenen Schmelze verknetet. Nach dem Abkühlen auf 20°C war die Schmelze fest und wurde in kleinere Bruchstücke zerteilt.

### Beispiel 3

250 g des gemäß Beispiel 1 erhaltenen Granulats wurden bei Raumtemperatur in 50 ml Wasser eingerührt. Nach wenigen Minuten bildete sich eine trübe Suspension, aus der sich kristallines Esupron als Bodensatz absetzte.

### Beispiel 4

Analog Beispiel 3 wurde das gemäß Beispiel 2 erhaltene Schmelzgranulat in Wasser eingerührt. Bereits nach wenigen Minuten bildete sich eine opaleszierende Lösung, aus der auch nach einer Stunde noch kein Esupron abgeschieden wurde.

## Patentansprüche

1. Mechanisch stabile pharmazeutische Darreichungsformen zur peroralen Verabreichung, enthaltend einen oder mehrerer Wirkstoffe, mindestens einen thermoplastisch verarbeitbaren matrixbildenden Hilfsstoff, der ausgewählt ist unter Homo- oder Copolymeren des N-Vinylpyrrolidons, Acrylatpolymeren vom Eudragit-Typ und Cellulosederivaten, und mehr als 10 und bis zu 40 Gew.-% einer oberflächenaktiven Substanz mit einem HLB-Wert von 2 bis 18, welche bei 20°C flüssig ist oder einen Tropfpunkt im Bereich von 20 bis 50°C aufweist, erhältlich durch Vermischen der Einsatzstoffe in der Schmelze ohne Zugabe von Lösungsmitteln und anschließender Formgebung.

2. Zubereitung nach Anspruch 1, enthaltend 15 bis 25 Gew.-% an oberflächenaktiver Substanz.

3. Zubereitungen nach Anspruch 1 oder 2, enthaltend oberflächenaktive Substanzen mit einem HLB-Wert von 10 bis 15.

4. Zubereitungen nach einem der Ansprüche 1 bis 3, enthaltend oberflächenaktive Substanzen mit einem Tropfpunkt im Bereich von 20 bis 40°C.

5. Zubereitungen nach einem der Ansprüche 1 bis 4, enthaltend oberflächenaktive Substanzen Macrogol-glycerol-hydroxystearat, Polyoxyethylenricinoleat-35 oder PEG-660-12-Hydroxystearinsäure.

6. Verfahren zur Herstellung mechanisch stabiler pharmazeutischer Darreichungsformen gemäß einem der Ansprüche 1 bis 5 nach einem Schmelzeverfahren, **dadurch gekennzeichnet, dass** man einen oder mehrere Wirkstoffe, mindestens einen thermoplastisch verarbeitbaren matrixbildenden Hilfsstoff sowie mehr als 10 und bis zu 40 Gew.-% einer oberflächenaktiven Substanz mit einem HLB-Wert von 2 bis 18, welche bei 20°C flüssig ist oder einen Tropfpunkt im Bereich von 20 bis 50°C aufweist, in der Schmelze zu einer homogenen Mischung verarbeitet und diese zu Darreichungsformen ausformt.

## Claims

1. Mechanically stable pharmaceutical formulations for peroral administration, containing one or more active substances, at least one thermoplastically workable matrix-forming adjuvant which is selected from among homo- or copolymers of N-vinylpyrrolidone, acrylate polymers of the Eudragit type and cellulose derivatives, and more than 10 and up to 40 wt.% of a surface-active substance with an HLB value of 2 to 18 which is liquid at 20°C or has a dropping point in the range from 20 to 50°C, obtainable by mixing the ingredients in a melt without addition of solvents, and subsequent shaping.

2. Preparation according to claim 1, containing 15 to 25 wt.% of surface active substance.

3. Preparations according to claim 1 or 2, containing surface active substances with an HLB value of 10 to 15.

4. Preparations according to one of claims 1 to 3, containing surface active substances with a dropping point in the range from 20 to 40°C.

5. Preparations according to one of claims 1 to 4, containing surface active substances Macrogol-glycerol-hydroxy stearate, polyoxyethylene ricinoleate-35 or PEG-660-12-hydroxy stearic acid.

6. Process for preparing mechanically stable pharmaceutical formulations according to one of claims 1 to 5 by a melting process, **characterised in that** one or more active substances, at least one thermoplastically workable matrix-forming adjuvant and more than 10 and up to 40 wt.% of a surface-active substance with an HLB value of 2 to 18 which is liquid at 20°C or has a dropping point in the range from 20 to 50°C, are worked in a melt to form a homogeneous mixture and the mixture is shaped into formulations.

## Revendications

1. Forme d'administration pharmaceutique mécaniquement stable pour administration par voie orale, qui comprend un ou plusieurs agents actifs, au moins un agent auxiliaire thermoplastique moulable formant une matrice, choisi parmi les mono- ou co-polymères de N-vinylpyrrolidone, les polymères d'acrylate de type Eudragit et les dérivés de cellulose, et entre 10 et 40 % en poids d'une substance tensioactive présentant une valeur HLB comprise entre 2 et 18, qui est liquide aux environs de 20 °C ou qui présente un point de goutte compris dans une plage de 20 à 50 °C, obtenue par mélange des produits de départ dans la masse en fusion sans ajout de solvant suivi d'une mise en forme.

2. Préparation selon la revendication 1, comprenant 15 à 25 % en poids d'une substance tensioactive.

3. Préparation selon la revendication 1 ou 2, comprenant une substance tensioactive présentant une valeur HLB comprise entre 10 et 15.

4. Préparation selon l'une quelconque des revendications 1 à 3, comprenant une substance tensioactive de point de goutte compris dans une plage de 20 à 40 °C.

5. Préparation selon l'une quelconque des revendications 1 à 4, comprenant en tant que substance tensioactive l'hydroxystéarate de Macrogol et de glycérol, le ricinoléate de polyoxyéthylène-35 ou le PEG-660-acide 12-hydroxystéarique.

6. Procédé de fabrication d'une forme d'administration pharmaceutique mécaniquement stable conforme à l'une des revendications 1 à 5 par un procédé en fusion, **caractérisé en ce que** à partir d'un ou plusieurs agents actifs, au moins un agent auxiliaire thermoplastique moulable formant une matrice, ainsi qu'une quantité comprise entre 10 et 40 % en poids d'une substance tensioactive présentant une valeur HLB comprise entre 2 et 18, qui est liquide aux environs de 20 °C ou qui présente un point de goutte compris dans une plage de 20 à 50 °C, on effectue un traitement dans la masse en fusion pour obtenir un mélange homogène, et un démoulage en la forme d'administration.
